(19) **Europäisches Patentamt / European Patent Office / Office européen des brevets**

(11) **EP 3 980 941 B1**

(12) **EUROPEAN PATENT SPECIFICATION**

(45) Date of publication and mention
of the grant of the patent:
**19.11.2025 Bulletin 2025/47**

(21) Application number: **20732418.7**

(22) Date of filing: **26.05.2020**

(51) International Patent Classification (IPC):
**G06N 3/0475** (2023.01)   **G06N 7/01** (2023.01)
**G06N 3/08** (2023.01)   **G16B 40/20** (2019.01)
**G06N 3/044** (2023.01)   **G06N 3/0464** (2023.01)

(52) Cooperative Patent Classification (CPC):
**G06N 3/08; G06N 3/0442; G06N 3/0464;
G06N 3/0475; G06N 3/09; G06N 7/01; G16B 40/20;
G06N 3/044**

(86) International application number:
**PCT/US2020/034475**

(87) International publication number:
**WO 2020/247200 (10.12.2020 Gazette 2020/50)**

(54) **LIKELIHOOD RATIOS FOR OUT-OF-DISTRIBUTION DETECTION**

WAHRSCHEINLICHKEITSVERHÄLTNISSE FÜR AUSSERVERTEILUNGSERKENNUNG

RAPPORTS DE VRAISEMBLANCE POUR DÉTECTION HORS-DISTRIBUTION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.06.2019 US 201962857774 P**

(43) Date of publication of application:
**13.04.2022 Bulletin 2022/15**

(73) Proprietor: **Google LLC
Mountain View, CA 94043 (US)**

(72) Inventors:
• **REN, Jie
  Mountain View, California 94043 (US)**
• **DILLON, Joshua Vincent
  Mountain View, California 94043 (US)**
• **LIU, Peter Junteng
  Mountain View, California 94043 (US)**
• **FERTIG, Emily Amanda
  Mountain View, California 94043 (US)**
• **LAKSHMINARAYANAN, Balaji
  Mountain View, California 94043 (US)**
• **SNOEK, Roland Jasper
  Mountain View, California 94043 (US)**
• **POPLIN, Ryan
  Mountain View, California 94043 (US)**
• **DEPRISTO, Mark Andrew
  Mountain View, California 94043 (US)**

(74) Representative: **Marks & Clerk GST
1 New York Street
Manchester M1 4HD (GB)**

(56) References cited:
• **ARNAB PODDAR ET AL: "Quality Measures for
  Speaker Verification with Short Utterances",
  ARXIV.ORG, CORNELL UNIVERSITY LIBRARY,
  201 OLIN LIBRARY CORNELL UNIVERSITY
  ITHACA, NY 14853, 29 January 2019 (2019-01-29),
  XP081009661**
• **DAN HENDRYCKS ET AL: "Deep Anomaly
  Detection with Outlier Exposure", ARXIV.ORG,
  CORNELL UNIVERSITY LIBRARY, 201 OLIN
  LIBRARY CORNELL UNIVERSITY ITHACA, NY
  14853, 11 December 2018 (2018-12-11),
  XP081018531**
• **ERIC NALISNICK ET AL: "Hybrid Models with
  Deep and Invertible Features", ARXIV.ORG,
  CORNELL UNIVERSITY LIBRARY, 201 OLIN
  LIBRARY CORNELL UNIVERSITY ITHACA, NY
  14853, 7 February 2019 (2019-02-07),
  XP081026578**

EP 3 980 941 B1

**(Cont. next page)**

- **AKOSUA BUSIA ET AL: "A deep learning approach to pattern recognition for short DNA sequences", BIORXIV, 28 January 2019 (2019-01-28), XP055725923, Retrieved from the Internet <URL:https://www.biorxiv.org/content/10.1101/353474v2.full.pdf> [retrieved on 20200828], DOI: 10.1101/353474**
- **AARON VAN DEN OORD ET AL: "Pixel Recurrent Neural Networks", 19 August 2016 (2016-08-19), pages 1 - 11, XP055435831, Retrieved from the Internet <URL:https://arxiv.org/pdf/1601.06759.pdf> [retrieved on 20171218]**
- **JIE REN ET AL: "Likelihood Ratios for Out-of-Distribution Detection", ARXIV.ORG, CORNELL UNIVERSITY LIBRARY, 201 OLIN LIBRARY CORNELL UNIVERSITY ITHACA, NY 14853, 7 June 2019 (2019-06-07), XP081546226**

## Description

RELATED APPLICATIONS

This application claims priority to United States Provisional Patent Application Number 62/857,774, filed June 5, 2019.

FIELD

The present disclosure relates generally to machine learning. More particularly, the present disclosure relates to systems and method for improved detection of out-of-distribution inputs. The term "in-distribution" is used to describe a dataset which is a sample from a certain data distribution. The distribution may be associated with a plurality of "in-distribution classes", which describe respective portions of the space of possible in-distribution datasets. By contrast, the term "out-of-distribution" (ODD) refers to a dataset which is not a sample from the distribution. The distribution may for example be a distribution of training examples which have been used to train a machine learning system. The datasets may be datasets obtained by observations and/or measurements of the real world, especially physical, biological, medical or chemical objects or events in the real world. For example, they may be datasets encoding nucleic acid sequences observed in the real-world, and/or datasets encoding sensor data (e.g. images) captured by one or more sensors (e.g. cameras including video cameras).

BACKGROUND

For many machine learning systems, being able to detect data that is anomalous or significantly different from that used in training can be critical to maintaining safe and reliable predictions. This is particularly important for deep neural network classifiers which have been shown to incorrectly classify OOD inputs into in-distribution classes with high confidence. This behavior can have serious consequences when the predictions inform real-world decisions such as medical diagnosis, e.g., falsely classifying a healthy sample as pathogenic or vice versa can have extremely high cost. As such, the importance of dealing with OOD inputs, also referred to as distributional shift, has been recognized as an important problem for AI safety.

One example sub-problem in which OOD detection is important is that of bacterial identification and many other types of medical diagnosis. For example, diagnosis and treatment of infectious diseases, such as sepsis, relies on the accurate detection of bacterial infections in blood. Several machine learning methods have been developed to perform bacteria identification by classifying known genomic sequences, including deep learning methods which are state-of-the-art.

However, even if neural network classifiers achieve high accuracy as measured through cross-validation, deploying them is challenging as real data is highly likely to contain genomes from unseen classes not present in the training data. In particular, different bacterial classes continue to be discovered gradually over the years and it is estimated that 60%-80% of genomic sequences belong to as yet unknown bacteria. Thus, training a classifier on existing bacterial classes and deploying it may result in OOD inputs being wrongly classified as one of the classes from the training data with high confidence. In addition, OOD inputs can also be the contaminations from the bacteria's host genomes such as human, plant, fungi, etc., which also need to be detected and excluded from predictions. Thus having a method for accurately detecting OOD inputs is critical to enable the practical application of machine learning methods to this important problem. In addition to this example sub-problem, the above-described dynamic and challenge is generalizable to many different scenarios/problems, including any scenario in which OOD inputs may be present and mis-classification of such OOD inputs would be problematic.

In the current state of the art, one popular strategy for detecting OOD inputs is to train a generative model on training data and use that to detect OOD inputs at test time. However, recent research has shown that deep generative models trained on image datasets can assign higher likelihood to OOD inputs (that is, the deep generative models can erroneously classify the OOD input as being in one of the in-distribution classes with higher likelihood than the deep generative models assign to an in-distribution input). Thus, these existing approaches may, in some scenarios, provide significantly erroneous and unreliable results. Arnab Poddar et al., "Quality Measures for Speaker Verification with Short Utterances" Arxiv.org (2019) proposes an automatic speaker verification system based on Gaussian mixture model-universal background model and i-vector. Dan Hendrycks et al., "Deep Anomaly Detection with Outlier Exposure" Arxiv.org (2018) is concerned with deep anomaly detection by training anomaly detectors against an auxiliary dataset of outliers. Eric Nalisnick et al., "Hybrid Models with Deep and Invertible Features" Arxiv.org (2019) discloses a neural hybrid model consisting of a linear model defined on a set of features computed by a deep, invertible transformation. Akosua Busia et al., "A deep learning approach to pattern recognition for short DNA sequences" Biorxiv.org (2019) describes a deep learning approach which trains a deep neural network (DNN) to predict database-derived labels directly from query sequences. Aaron Van Den Oord et al., "Pixel Recurrent Neural Networks" Arxiv.org (2016) presents a deep neural network that sequentially predicts the pixels in an image along the two spatial dimensions.

SUMMARY

Aspects and advantages of embodiments of the present disclosure will be set forth in part in the following description, or can be learned from the description, or can be learned through practice of the embodiments. According to a first aspect there is provided a computing system as defined by claim 1 that performs genomic sequence classification. According to another aspect there is provided a computing system as defined by claim 2 that performs image classification. According to a further aspect there is provided a computer-implemented method as defined by claim 8 to perform genomic sequence classification. According to yet a further aspect there is provided a computer-implemented method as defined by claim 9 to perform image classification. According to a further aspect there is provided one or more non-transitory computer-readable media as defined by claim 15.

These and other features, aspects, and advantages of various embodiments of the present disclosure will become better understood with reference to the following description and appended claims. The accompanying drawings, which are incorporated in and constitute a part of this specification, illustrate example embodiments of the present disclosure and, together with the description, serve to explain the related principles.

BRIEF DESCRIPTION OF THE DRAWINGS

Detailed discussion of embodiments directed to one of ordinary skill in the art is set forth in the specification, which makes reference to the appended figures, in which:

Figure 1A depicts a block diagram of an example computing system according to example embodiments of the present disclosure.
Figure 1B depicts a block diagram of an example computing device according to example embodiments of the present disclosure.
Figure 1C depicts a block diagram of an example computing device according to example embodiments of the present disclosure.
Figure 2 depicts a block diagram of an example OOD detection and classification system according to example embodiments of the present disclosure.
Figure 3 depicts a flow chart diagram of an example method to generate an OOD detection system according to example embodiments of the present disclosure.

Reference numerals that are repeated across plural figures are intended to identify the same features in various implementations.

DETAILED DESCRIPTION

Overview

Generally, the present disclosure is directed to systems and method to perform improved detection of out-of-distribution (OOD) inputs. In particular, current deep generative model-based approaches for OOD detection are significantly negatively affected by and struggle to distinguish population level background statistics from semantic content relevant to the in-distribution examples (that is, to distinguish between portions of in-distribution inputs which are relevant for or specific to classifying the input into in a corresponding one of a plurality of classes ("sematic content"), and portions of the in-distribution inputs which are not relevant for or less specific to classification into such classes and which may be in common with OOD inputs ("background statistics")). In fact, such approaches have even been experimentally observed to assign higher likelihood to OOD inputs, which is opposite to the desired behavior. To resolve this problem, the present disclosure proposes a likelihood ratio method for deep generative models which effectively corrects for these confounding background statistics.

Specifically, the proposed likelihood ratio method can leverage both a semantic model that learns the semantic features of the in-distribution training examples and a background model that learns to correct for the background statistics of the training examples. In particular, the semantic model can be trained on a set of in-distribution training examples while the background model can be trained on a set of background training examples, where at least some of the background training examples are generated through corruption of the semantic content of the in-distribution training examples (e.g., through perturbation of the in-distribution training example).

After training, OOD detection for a given data input can be performed based on a likelihood ratio value generated from the respective likelihood values output by the semantic model and the background model for the data input. Use of the background model in this fashion can enhance the in-distribution specific features for OOD detection, leading to state-of-the-art performance of OOD detection.

The proposed approach has been experimentally shown to significantly outperform the raw likelihood on OOD detection for deep generative models on image datasets. As examples, United States Provisional Patent Application Number 62/857,774, and Jie Ren et al, "Likelihood Ratios for Out-of-distribution Detection", arXiv: 1906.02845, contain example experimental results which demonstrate the efficacy of the proposed approach in improving OOD detection.

The systems and methods of the present disclosure provide a number of technical effects and benefits. As one example, the proposed technique effectively corrects for the background components, thereby significantly improving the accuracy of OOD detection on multiple input data modalities. By improving OOD detection, the underlying accuracy of the system can be improved, for example, by preventing OOD inputs from being mis-classified by a downstream classifier. Thus, the proposed technique can improve the accuracy of a system that performs classification of inputs, leading to more improved system performance.

As one example, in a system that performs bacterial identification or other types of medical diagnosis, the number of inaccurate diagnoses (e.g., false positives) can be reduced, thereby improving the efficacy of medical care supplied based on the provided diagnoses and reducing costs associated with unnecessary and/or incorrect medical treatments. Thus, improved healthcare outcomes can be achieved at a reduced cost.

As another example technical effect and benefit, by improving OOD detection, the deployment of machine learning systems can be extended to new uses and scenarios in which classification accuracy is critical. Thus, by reducing mis-classification of OOD inputs, AI safety can be improved, allowing the benefits of machine learning systems to be brought to bear upon new problem domains or existing challenges. Furthermore, public confidence in machine learning applications can be increased.

As another example technical effect and benefit, by using the improved OOD detection systems of the present disclosure to screen inputs prior to processing by downstream system components (e.g., a classifier), the unnecessary and undesirable processing of OOD inputs by such downstream components can be reduced. Thus, computing resources such as processor usage, memory usages, network bandwidth, etc., can be conserved. Stated differently, by identifying and screening OOD inputs at an earlier stage of the processing pipeline, the wasteful application of downstream resources to such OOD inputs can be reduced.

Example implementations of the techniques described herein will now be discussed in greater detail.

<u>Example Notation and Problem Statement</u>

Suppose there exists an in-distribution dataset $\mathcal{D}$ of (x, y) pairs sampled from the distribution p*(x, y), where x is the extracted feature vector or raw input and $y \in \mathcal{Y}$ := $\{1, ..., k, ..., K\}$ is the label assigning membership to one of $K$ in-distribution classes. For simplicity, this discussion assumes inputs to be discrete, e.g., $x_d \in \{A, C, G, T\}$ for genomic sequences and $x_d \in \{0, ...,255\}$ for images, where $d$ is an integer, and $x_d$ denotes the $d$-th component of x.

In general, OOD inputs are samples (x, y) generated from an underlying distribution other than p*(x, y). As used herein, an input (x, y) is considered to be OOD if $y \notin \mathcal{Y}$: that is, the class $y$ does not belong to one of the $K$ in-distribution classes. One goal of an OOD detection system is to accurately detect if an input x is OOD or not.

<u>Failure of Existing Generative Model Approaches</u>

Certain existing methods involve computing statistics using the predictions of (ensembles of) discriminative classifiers trained on in-distribution data, e.g., taking the confidence or entropy of the predictive distribution $p(y|x)$.

An alternative is to use generative model-based methods, which are appealing as they do not require labeled data and directly model the input distribution. These methods fit a generative model $p(x)$ to the input data, and then evaluate the likelihood of new inputs under that model (i.e. the likelihood that a new input is drawn from $p(x)$). However, recent work has highlighted significant issues with this approach for OOD detection on images, showing that deep generative models such as Glow and PixelCNN sometimes assign higher likelihoods to OOD than in-distribution inputs.

Similarly, example experiments contained in the United States Provisional Patent Application Number 62/857,774, and Jie Ren et al, "Likelihood Ratios for Out-of-distribution Detection", arXiv: 1906.02845, demonstrate that density estimation-based methods exhibit similar failures for OOD detection in genomics. In particular, these example experiments showed that the log-likelihood under the model is heavily affected by a sequence's *GC-content*. GC-content is defined as the percentage of bases that are either G or C, and is used widely in genomic studies as a basic statistic for describing overall genomic composition, and studies have shown that bacteria have an astonishing diversity of genomic GC-content, from 16.5% to 75%.

Bacteria from similar groups tend to have similar GC-content at the population level, but they also have characteristic biological patterns that can distinguish them well from each other. The confounding effect of GC-content makes the likelihood less reliable as a score for OOD detection, because an OOD input may result in a higher likelihood than an in-distribution input, because it has high GC-content and not necessarily because it contains characteristic patterns specific to the in-distribution bacterial classes.

Thus, more generally, it may be said that the failure of existing generative model approaches is attributable to the inability of the generative model to learn to distinguish background content in the new input to be analyzed from semantic content included in the in-distribution examples.

Example Likelihood Ratio for OOD Detection

This section first provides a high level conceptual overview and then describes examples of how to adapt it to deep generative models.

Example High Level Conceptual Overview

Assume that an input x is composed of two components, (1) a background component characterized by population level background statistics, and (2) a semantic component characterized by patterns specific to the in-distribution data. For example, images can be modeled as backgrounds plus objects; text can be considered as a combination of high frequency stop words plus semantic words; genomes can be modeled as background sequences plus motifs; and/or other modalities of data can be bifurcated into semantic and background content.

More formally, for a D-dimensional input $x = x_1, ..., x_D$, aspects of the present disclosure assume that there exists an unobserved variable $z = z_1, ..., z_D$, where $z_d \in \{B, S\}$ indicates if the $d$th dimension of the input $x_d$ is generated from the Background component or the Semantic component. Grouping the semantic and background parts, the input can be factored as $x = \{x_B, x_S\}$ where $x_B = \{x_d | z_d = B, d = 1, ..., D\}$. For simplicity, assume that the background and semantic components are generated independently. The likelihood can be then decomposed as follows,

$$p(\mathbf{x}) = p(\mathbf{x}_B)p(\mathbf{x}_S). \quad (1)$$

Existing approaches to training and evaluating deep generative models do not distinguish between these two terms in the likelihood. However, the present disclosure recognizes that it may be preferred to use just the semantic likelihood $p(x_S)$ to avoid the likelihood term being dominated by the background term (e.g., such that the likelihood is similar if the input is an OOD input with the same background but different semantic component). In practice, only x is observed, and it is not always easy to split an input into background and semantic parts $\{x_B, x_S\}$.

Thus, as a practical alternative, the present disclosure proposes training a background model by perturbing inputs. Adding the right amount of perturbations to inputs can corrupt the semantic structure in the data, and hence the model trained on perturbed inputs captures only the population level background statistics.

More particularly, assume that $p_\theta(\cdot)$ is a model trained using in-distribution data, and $p_{\theta_0}(\cdot)$ is a background model that captures general background statistics. The present disclosure provides a likelihood ratio statistic that is defined as

$$\mathrm{LLR}(\mathbf{x}) = \log \frac{p_\theta(\mathbf{x})}{p_{\theta_0}(\mathbf{x})} = \log \frac{p_\theta(\mathbf{x}_B)\, p_\theta(\mathbf{x}_S)}{p_{\theta_0}(\mathbf{x}_B)\, p_{\theta_0}(\mathbf{x}_S)}, \quad (2)$$

where the factorization from Equation 1 is used.

Assume that (i) both models capture the background information equally well, that is $P_\theta(X_B) \approx p_{\theta_0}(X_B)$ and (ii) $p_\theta(x_S)$ is more peaky (e.g., predicts larger and more frequent likelihoods) than $p_{\theta_0}(x_S)$ as the former is trained on data containing semantic information, while the latter model $\theta_0$ is trained using data with noise perturbations. Then, the likelihood ratio can be approximated as

$$\mathrm{LLR}(\mathbf{x}) \approx \log p_\theta(\mathbf{x}_S) - \log p_{\theta_0}(\mathbf{x}_S). \quad (3)$$

Thus, by taking the ratio, the likelihood for the background component $X_B$ is cancelled out, and only the likelihood for the semantic component $x_S$ remains. As such, the proposed method produces a background contrastive score that captures the significance of the semantics compared with the background model.

Example Application of Likelihood Ratio to Auto-Regressive Models

Auto-regressive models are one of the popular choices for generating images and sequence data such as genomics, drug molecules, audio, and text. In auto-regressive models, the log-likelihood of an input can be expressed as $\log p_\theta(\mathbf{x}) = \sum_{d=1}^{D} \log p_\theta(x_d|\mathbf{x}_{<d})$, where $\mathbf{x}_{<d} = x_1 \ldots x_{d-1}$. Decomposing the log-likelihood into background and semantic parts, we have

$$\log p_\theta(\mathbf{x}) = \sum_{d:x_d \in \mathbf{x}_B} \log p_\theta(x_d|\mathbf{x}_{<d}) + \sum_{d:x_d \in \mathbf{x}_S} \log p_\theta(x_d|\mathbf{x}_{<d}). \quad (4)$$

A similar auto-regressive decomposition can be used for the background model $p_{\theta_0}(\mathbf{x})$ as well. Assuming that both the models capture the background information equally well,

$$\sum_{d:x_d \in \mathbf{x}_B} \log p_\theta(x_d|\mathbf{x}_{<d}) \approx \sum_{d:x_d \in \mathbf{x}_B} \log p_{\theta_0}(x_d|\mathbf{x}_{<d}),$$

the likelihood ratio is approximated as

$$\mathrm{LLR}(\mathbf{x}) \approx \sum_{d:x_d \in \mathbf{x}_S} \log p_\theta(x_d|\mathbf{x}_{<d}) - \sum_{d:x_d \in \mathbf{x}_S} \log p_{\theta_0}(x_d|\mathbf{x}_{<d})$$

$$= \sum_{d:x_d \in \mathbf{x}_S} \log \frac{p_\theta(x_d|\mathbf{x}_{<d})}{p_{\theta_0}(x_d|\mathbf{x}_{<d})}. \quad (5)$$

Example Techniques to Train the Background Model

Any number of different techniques can be performed to perturb the in-distribution training examples to corrupt their semantic data, thereby generating background training examples.

As one example technique, noise can be added to the training example to perturb it. As one specific example, if the training example is discrete (e.g., $x_d \in \mathcal{A}$) (e.g., $\mathcal{A} = \{A, C, G, T\}$ for genomic sequences, $\mathcal{A} = \{0, ...,255\}$ for images, $\mathcal{A}$ = a dictionary of words, graphemes, phonemes, and/or n-grams for textual content, $\mathcal{A}$ = a set of possible amplitudes and/or frequencies for audio content, etc.), noise can be added by performing the follow operations:

1: Generate a $D$-dimensional vector $v = v_1 \ldots , v_D$, where $v_d \in \{0,1\}$ are independent and identically distributed according to a Bernoulli distribution with rate $\mu$.
2: For index $d \in \{1, \ldots , D\}$ do
3: If $v_d = 1$ then
4: Sample $\bar{x}_d$ from the set $\mathcal{A}$ with equal probability.
5: Else
6: Set $\bar{x}_d = x_d$.
7: End if
8: End for

Thus, as one example technique, in implementations in which the training examples include a sequence of DNA characters, a computing system can add perturbations to the input data by randomly selecting positions in $x_1 \ldots x_D$ following an independent and identical Bernoulli distribution with rate $\mu$ and substituting the original character with one of the other characters with equal probability. The procedure is inspired by genetic mutations.

The rate $\mu$ is a hyperparameter and can be easily tuned using a small amount of a validation OOD dataset (e.g., which is different from the actual OOD dataset of interest). In the case where a validation OOD dataset is not available, $\mu$ can also be tuned using simulated OOD data. In practice, $\mu \in [0.1, 0.2]$ has been shown to achieve good performance empirically.

Semantic content included in the input image can be explicitly recognized (e.g., using a semantic segmentation model) and destroyed (e.g., removed from the image and replaced with noise)

In addition to perturbations of the input data, other techniques can improve model generalization and prevent model memorization. As one example, adding $L_2$ regularization with coefficient $\lambda$ to model weights can help to train a good background model. In fact, adding noise to the input is equivalent to adding $L_2$ regularization to the model weights under some conditions. As another example, performing early stopping of the training of the background model (e.g., without or without the perturbation and/or additional regularization terms) can result in a background model that effectively cancels background statistics. Besides the methods above, adding other types of noise or regularization methods would show a similar effect.

Example Devices and Systems

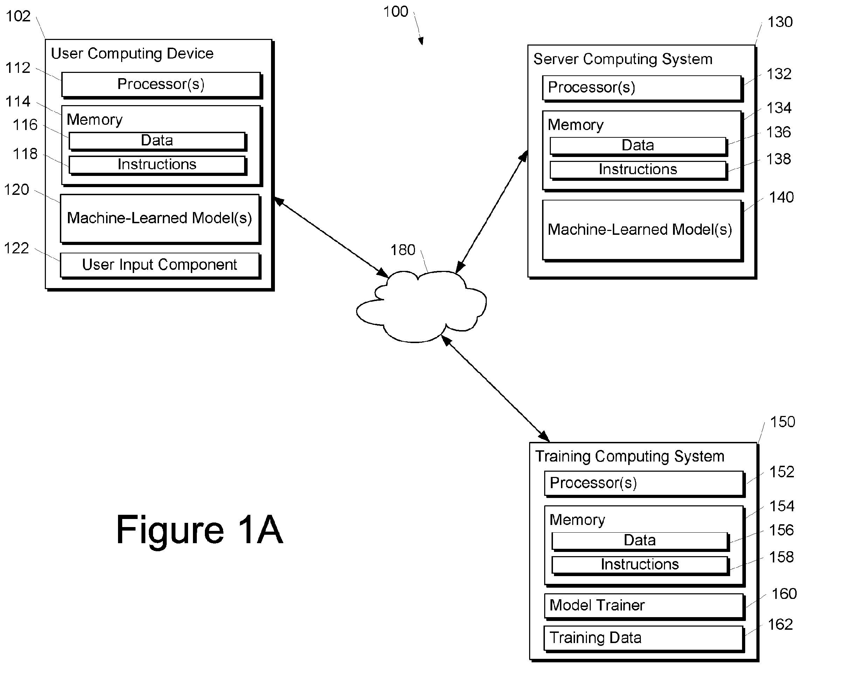

Figure 1A depicts a block diagram of an example computing system 100 that performs OOD detection according to example embodiments of the present disclosure. The system 100 includes a user computing device 102, a server computing system 130, and a training computing system 150 that are communicatively coupled over a network 180.

The user computing device 102 can be any type of computing device, such as, for example, a personal computing device (e.g., laptop or desktop), a mobile computing device (e.g., smartphone or tablet), a gaming console or controller, a wearable computing device, an embedded computing device, or any other type of computing device.

The user computing device 102 includes one or more processors 112 and a memory 114. The one or more processors 112 can be any suitable processing device (e.g., a processor core, a microprocessor, an ASIC, a FPGA, a controller, a microcontroller, etc.) and can be one processor or a plurality of processors that are operatively connected. The memory 114 can include one or more non-transitory computer-readable storage mediums, such as RAM, ROM, EEPROM, EPROM, flash memory devices, magnetic disks, etc., and combinations thereof. The memory 114 can store data 116 and instructions 118 which are executed by the processor 112 to cause the user computing device 102 to perform operations.

In some implementations, the user computing device 102 can store or include one or more machine-learned models 120. For example, the machine-learned models 120 can be or can otherwise include various machine-learned models such as neural networks (e.g., deep neural networks) or other types of machine-learned models, including non-linear models and/or linear models. Neural networks can include feed-forward neural networks, recurrent neural networks (e.g., long short-term memory recurrent neural networks), convolutional neural networks or other forms of neural networks.

In some implementations, the one or more machine-learned models 120 can be received from the server computing system 130 over network 180, stored in the user computing device memory 114, and then used or otherwise implemented by the one or more processors 112. In some implementations, the user computing device 102 can implement multiple parallel instances of a single machine-learned model 120 (e.g., to perform parallel OOD detection across multiple instances of data inputs). In some implementations, the processors 112 can implement the instructions 118 to determine a likelihood ratio value for a data input based on outputs from the model(s) 120 and detect whether the data input is OOD based on the likelihood ratio value.

Additionally or alternatively, one or more machine-learned models 140 can be included in or otherwise stored and implemented by the server computing system 130 that communicates with the user computing device 102 according to a client-server relationship. For example, the machine-learned models 140 can be implemented by the server computing system 140 as a portion of a web service (e.g., an OOD detection service). Thus, one or more models 120 can be stored and implemented at the user computing device 102 and/or one or more models 140 can be stored and implemented at the server computing system 130.

The user computing device 102 can also include one or more user input component 122 that receives user input. For example, the user input component 122 can be a touch-sensitive component (e.g., a touch-sensitive display screen or a touch pad) that is sensitive to the touch of a user input object (e.g., a finger or a stylus). The touch-sensitive component can serve to implement a virtual keyboard. Other example user input components include a microphone, a traditional keyboard, or other means by which a user can provide user input.

The server computing system 130 includes one or more processors 132 and a memory 134. The one or more processors 132 can be any suitable processing device (e.g., a processor core, a microprocessor, an ASIC, a FPGA, a controller, a microcontroller, etc.) and can be one processor or a plurality of processors that are operatively connected. The memory 134 can include one or more non-transitory computer-readable storage mediums, such as RAM, ROM, EEPROM, EPROM, flash memory devices, magnetic disks, etc., and combinations thereof. The memory 134 can store data 136 and instructions 138 which are executed by the processor 132 to cause the server computing system 130 to perform operations.

In some implementations, the server computing system 130 includes or is otherwise implemented by one or more server computing devices. In instances in which the server computing system 130 includes plural server computing devices, such server computing devices can operate according to sequential computing architectures, parallel computing architectures, or some combination thereof.

As described above, the server computing system 130 can store or otherwise include one or more machine-learned models 140. For example, the models 140 can be or can otherwise include various machine-learned models. Example machine-learned models include neural networks or other multi-layer non-linear models. Example neural networks include feed forward neural networks, deep neural networks, recurrent neural networks, and convolutional neural networks.

In some implementations, the processors 132 can implement the instructions 138 to determine a likelihood ratio value for a data input based on outputs from the model(s) 140 and detect whether the data input is OOD based on the likelihood ratio value.

The user computing device 102 and/or the server computing system 130 can train the models 120 and/or 140 via interaction with the training computing system 150 that is communicatively coupled over the network 180. The training computing system 150 can be separate from the server computing system 130 or can be a portion of the server computing system 130.

The training computing system 150 includes one or more processors 152 and a memory 154. The one or more processors 152 can be any suitable processing device (e.g., a processor core, a microprocessor, an ASIC, a FPGA, a controller, a microcontroller, etc.) and can be one processor or a plurality of processors that are operatively connected. The memory 154 can include one or more non-transitory computer-readable storage mediums, such as RAM, ROM, EEPROM, EPROM, flash memory devices, magnetic disks, etc., and combinations thereof. The memory 154 can store data 156 and instructions 158 which are executed by the processor 152 to cause the training computing system 150 to perform operations. In some implementations, the training computing system 150 includes or is otherwise implemented by one or more server computing devices.

The training computing system 150 can include a model trainer 160 that trains the machine-learned models 120 and/or 140 stored at the user computing device 102 and/or the server computing system 130 using various training or learning techniques, such as, for example, backwards propagation of errors. In some implementations, performing backwards propagation of errors can include performing truncated backpropagation through time. The model trainer 160 can perform a number of generalization techniques (e.g., weight decays, dropouts, etc.) to improve the generalization capability of the models being trained.

In particular, the model trainer 160 can train the machine-learned models 120 and/or 140 based on a set of training data 162. The training data 162 can include, for example, image data, textual data, audio data, genomic data, sensor data, etc.

In some implementations, the model trainer 160 can perform some or all of the data perturbation techniques described herein.

In some implementations, if the user has provided consent, the training examples can be provided by the user computing device 102. Thus, in such implementations, the model 120 provided to the user computing device 102 can be trained by the training computing system 150 on user-specific data received from the user computing device 102. In some instances, this process can be referred to as personalizing the model.

The model trainer 160 includes computer logic utilized to provide desired functionality. The model trainer 160 can be implemented in hardware, firmware, and/or software controlling a general purpose processor. For example, in some implementations, the model trainer 160 includes program files stored on a storage device, loaded into a memory and executed by one or more processors. In other implementations, the model trainer 160 includes one or more sets of computer-executable instructions that are stored in a tangible computer-readable storage medium such as RAM hard disk or optical or magnetic media.

The network 180 can be any type of communications network, such as a local area network (e.g., intranet), wide area network (e.g., Internet), or some combination thereof and can include any number of wired or wireless links. In general, communication over the network 180 can be carried via any type of wired and/or wireless connection, using a wide variety of communication protocols (e.g., TCP/IP, HTTP, SMTP, FTP), encodings or formats (e.g., HTML, XML), and/or protection schemes (e.g., VPN, secure HTTP, SSL).

Figure 1A illustrates one example computing system that can be used to implement the present disclosure. Other computing systems can be used as well. For example, in some implementations, the user computing device 102 can include the model trainer 160 and the training dataset 162. In such implementations, the models 120 can be both trained and used locally at the user computing device 102. In some of such implementations, the user computing device 102 can implement the model trainer 160 to personalize the models 120 based on user-specific data.

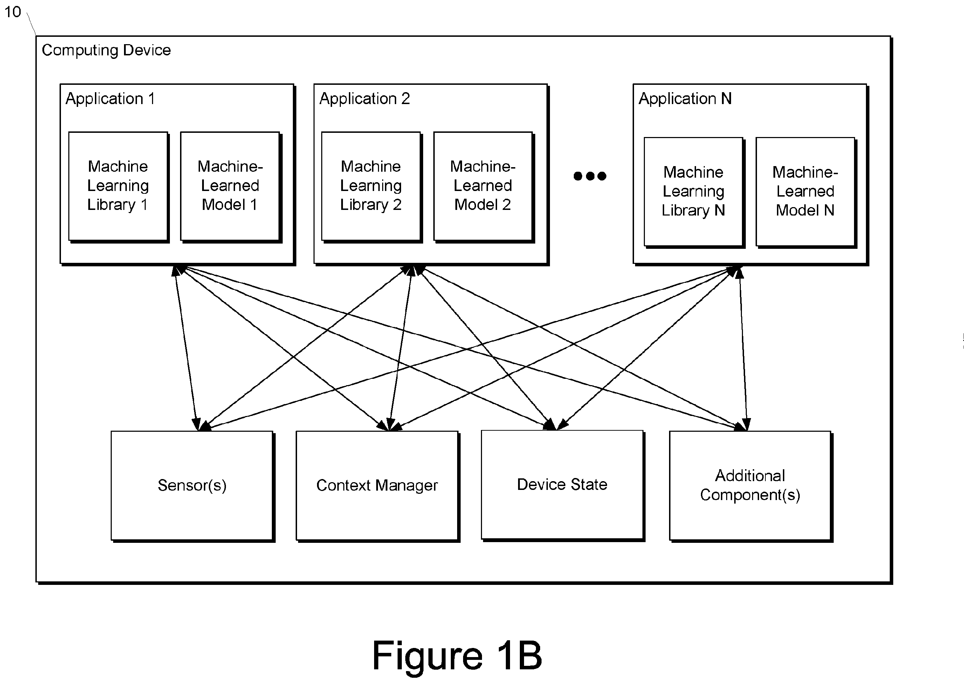

Figure 1B depicts a block diagram of an example computing device 10 that performs according to example embodiments of the present disclosure. The computing device 10 can be a user computing device or a server computing device.

The computing device 10 includes a number of applications (e.g., applications 1 through N). Each application contains its own machine learning library and machine-learned model(s). For example, each application can include a machine-learned model. Example applications include a text messaging application, an email application, a dictation application, a virtual keyboard application, a browser application, etc.

As illustrated in Figure 1B, each application can communicate with a number of other components of the computing device, such as, for example, one or more sensors, a context manager, a device state component, and/or additional components. In some implementations, each application can communicate with each device component using an API (e.g., a public API). In some implementations, the API used by each application is specific to that application.

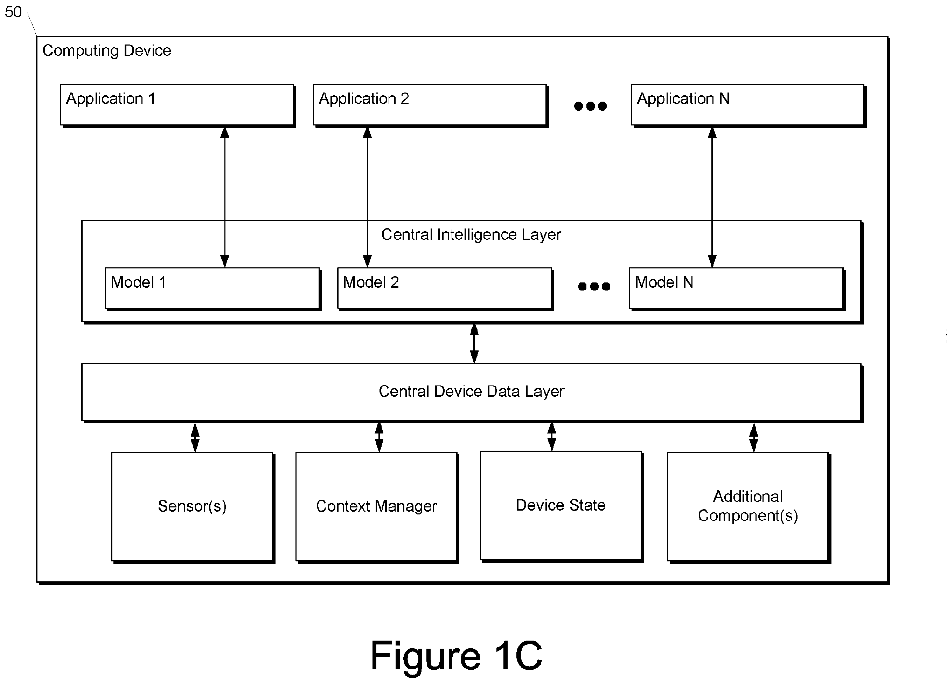

Figure 1C depicts a block diagram of an example computing device 50 that performs according to example embodiments of the present disclosure. The computing device 50 can be a user computing device or a server computing device.

The computing device 50 includes a number of applications (e.g., applications 1 through N). Each application is in communication with a central intelligence layer. Example applications include a text messaging application, an email application, a dictation application, a virtual keyboard application, a browser application, etc. In some implementations, each application can communicate with the central intelligence layer (and model(s) stored therein) using an API (e.g., a common API across all applications).

The central intelligence layer includes a number of machine-learned models. For example, as illustrated in Figure 1C, a respective machine-learned model (e.g., a model) can be provided for each application and managed by the central intelligence layer. In other implementations, two or more applications can share a single machine-learned model. For example, in some implementations, the central intelligence layer can provide a single model (e.g., a single model) for all of the applications. In some implementations, the central intelligence layer is included within or otherwise implemented by an operating system of the computing device 50.

The central intelligence layer can communicate with a central device data layer. The central device data layer can be a centralized repository of data for the computing device 50. As illustrated in Figure 1C, the central device data layer can communicate with a number of other components of the computing device, such as, for example, one or more sensors, a context manager, a device state component, and/or additional components. In some implementations, the central device data layer can communicate with each device component using an API (e.g., a private API).

Example Model Arrangements

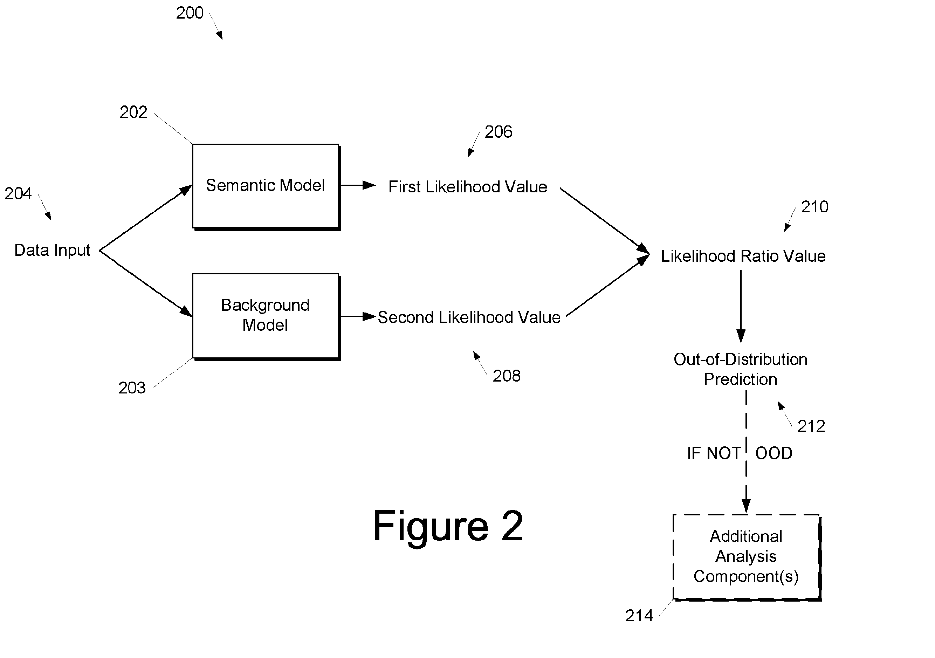

Figure 2 depicts a block diagram of an OOD detection system 200 according to example embodiments of the present disclosure. The detection system includes a semantic model 202 and a background model 203.

The semantic model 202 has been trained on a set of in-distribution training data that includes a plurality of in-distribution training examples. The semantic model 202 is configured to receive and process a data input 204 to generate a first likelihood value 206 for the data input 204.

The background model 203 has been trained on a set of background training data comprising a plurality of background training examples. The one or more background training examples of the plurality of background training examples has been generated through perturbation of one or more in-distribution training examples of the plurality of in-distribution training examples. The background model 203 is configured to receive and process the data input 204 to generate a second likelihood value 208 for the data input 204.

Both of the semantic model 202 and the background model 203 are a generative model. In some implementations, one or both of the semantic model 202 and the background model 203 can be an autoregressive model. In some implementations, one or both of the semantic model 202 and the background model 203 can be a neural network such as a recurrent neural network and/or a convolutional neural network.

The OOD detection system 200 (e.g., as implemented by one or more computing devices) determines a likelihood ratio value 210 for the data input 204 based at least in part on the first likelihood value 206 generated by semantic model 202 and the second likelihood value 208 generated by the background model 203. The system 200 generates, based at least in part on the likelihood ratio value 210, an OOD prediction 212 that indicates whether the data input 204 is out-of-distribution.

In some implementations, determining the likelihood ratio value 210 for the data input 204 can include determining a logarithm of the first likelihood value 206 divided by the second likelihood value 208. In some implementations, generating the prediction 212 based at least in part on the likelihood ratio value 210 can include comparing the likelihood ratio value to a threshold value and predicting that the data input 204 is OOD when the likelihood ratio value 210 is less than the threshold value. The threshold value can be a hyperparameter that is user-specified or learned by the system.

In some implementations, when it is predicted that the data input 204 is not OOD, the system 200 can provide the data input 204 to one or more additional analysis components 214 such as, for example, a machine-learned classifier model for classification relative to a plurality of in-distribution classes.

Example Methods

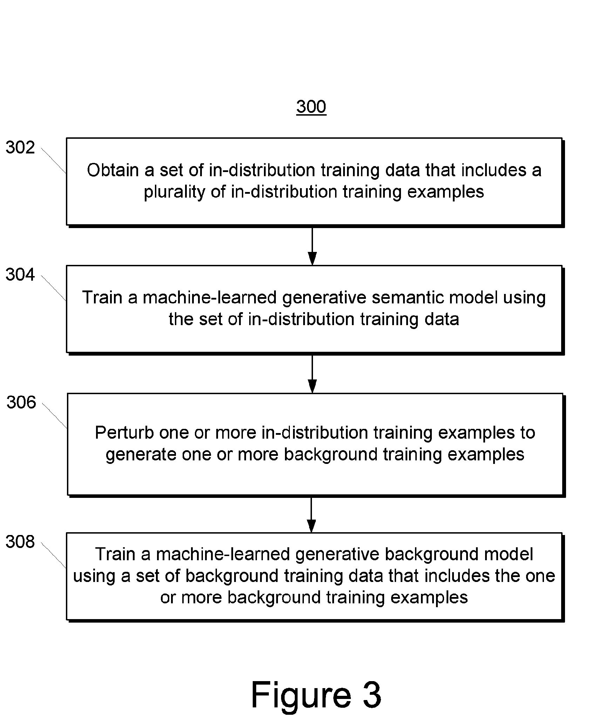

Figure 3 depicts a flow chart diagram of an example method to perform according to example embodiments of the present disclosure.

At 302, a computing system obtains a set of in-distribution training data that includes a plurality of in-distribution training examples. The in-distribution training examples are images or genomic data.

At 304, the computing system trains a machine-learned generative semantic model using the set of in-distribution training data.

At 306, the computing system perturbs one or more in-distribution training examples of the plurality of in-distribution training examples to generate one or more background training examples.

In some implementations, the perturbation of the one or more in-distribution training examples to generate the one or more background training examples can include adding noise to the one or more in-distribution training example.

In some implementations, each of the plurality of in-distribution training examples can include semantic content related to at least one of a plurality of in-distribution classes associated with the set of in-distribution training data; and the perturbation of the one or more in-distribution training examples to generate the one or more background training examples can include corrupting the semantic content included in the one or more in-distribution training examples.

In some implementations, each of the one or more in-distribution training examples includes a respective genomic sequence of DNA characters and the perturbation of the one or more in-distribution training examples to generate the one or more background training examples can include randomly mutating one or more characters of each respective genomic sequence of DNA characters to one or more alternative DNA characters.

In some implementations, each of the one or more in-distribution training examples comprises a respective image comprising a plurality of pixels and the perturbation of the one or more in-distribution training examples to generate the one or more background training examples can include, for each respective image, randomly changing respective pixel values for one or more of the plurality of pixels.

At 308, the computing system can train a machine-learned generative background model using a set of background training data that that includes the one or more background training examples.

In some implementations, the machine-learned generative semantic model can be trained at 304 using a first loss function and the machine-learned generative background model can be trained at 308 using a second loss function that is equal to the first loss function plus an additional L2 regularization term.

In some implementations, the machine-learned generative semantic model can be trained at 304 for a first number of training iterations and the machine-learned generative background model can be trained at 308 for a second number of iterations that is less than the first number of iterations. For example, the second number of iterations can be less than 50%, 60%, 70%, 80% or 90% of the first number of training iterations.

Additional Disclosure

The technology discussed herein makes reference to servers, databases, software applications, and other computer-based systems, as well as actions taken and information sent to and from such systems. The inherent flexibility of computer-based systems allows for a great variety of possible configurations, combinations, and divisions of tasks and functionality between and among components. For instance, processes discussed herein can be implemented using a single device or component or multiple devices or components working in combination. Databases and applications can be implemented on a single system or distributed across multiple systems. Distributed components can operate sequentially or in parallel.

**Claims**

1. A computing system that performs genomic sequence classification for bacterial identification or medical diagnosis, the computing system comprising:

   one or more processors; and
   one or more non-transitory computer-readable media that collectively store:

   a machine-learned generative semantic model (202) trained on a set of in-distribution training data comprising a plurality of in-distribution training examples which are samples of a distribution, the machine-learned generative semantic model configured to receive and process a data input to generate a first likelihood value (206) for the data input, the first likelihood value being a first indication of the likelihood that the data input is a sample from the distribution;

a machine-learned generative background model (203) trained on a set of background training data comprising a plurality of background training examples, one or more background training examples of the plurality of background training examples generated through perturbation of one or more in-distribution training examples of the plurality of in-distribution training examples (306), the machine-learned generative background model configured to receive and process the data input to generate a second likelihood value (208) for the data input, the second likelihood value being a second indication of the likelihood that the data input is a sample from a background distribution; and

instructions that, when executed by the one or more processors, cause the computing system to perform operations comprising:

determining a likelihood ratio value (210) for the data input based at least in part on the first likelihood value generated by the machine-learned generative semantic model and the second likelihood value generated by the machine-learned generative background model;

predicting whether the data input is not a sample from the distribution (212) based at least in part on the likelihood ratio value; and

when it is predicted that the data input is a sample from the distribution, providing the data input to a machine-learned classifier model for classification relative to a plurality of in-distribution classes;

wherein the data input is a genomic sequence, and

each of the one or more in-distribution training examples comprises a respective genomic sequence of DNA characters and the perturbation of the one or more in-distribution training examples to generate the one or more background training examples comprises randomly mutating one or more characters of each respective genomic sequence of DNA characters to one or more alternative DNA characters.

2. A computing system that performs image classification, the computing system comprising:

one or more processors; and

one or more non-transitory computer-readable media that collectively store:

a machine-learned generative semantic model (202) trained on a set of in-distribution training data comprising a plurality of in-distribution training examples which are samples of a distribution, the machine-learned generative semantic model configured to receive and process a data input to generate a first likelihood value (206) for the data input, the first likelihood value being a first indication of the likelihood that the data input is a sample from the distribution;

a machine-learned generative background model (203) trained on a set of background training data comprising a plurality of background training examples, one or more background training examples of the plurality of background training examples generated through perturbation of one or more in-distribution training examples of the plurality of in-distribution training examples (306), the machine-learned generative background model configured to receive and process the data input to generate a second likelihood value (208) for the data input, the second likelihood value being a second indication of the likelihood that the data input is a sample from a background distribution; and

instructions that, when executed by the one or more processors, cause the computing system to perform operations comprising:

determining a likelihood ratio value (210) for the data input based at least in part on the first likelihood value generated by the machine-learned generative semantic model and the second likelihood value generated by the machine-learned generative background model;

predicting whether the data input is not a sample from the distribution (212) based at least in part on the likelihood ratio value; and

when it is predicted that the data input is a sample from the distribution, providing the data input to a machine-learned classifier model for classification relative to a plurality of in-distribution classes;

wherein the data input is an image, and

each of the one or more in-distribution training examples comprises a respective image comprising a plurality of pixels and the perturbation of the one or more in-distribution training examples to generate the one or more background training examples comprises, for each respective image, randomly changing respective pixel values for one or more of the plurality of pixels.

3. The computing system of claim 1 or 2, wherein determining the likelihood ratio (210) value for the data input comprises determining a logarithm of the first likelihood value (206) divided by the second likelihood value (208); and/or wherein predicting whether the data input is not a sample from the distribution based at least in part on the likelihood ratio value (210) comprises:

comparing the likelihood ratio value to a threshold value; and
predicting that the data input is not a sample from the distribution when the likelihood ratio value is less than the threshold value.

4. The computing system of any preceding claim, wherein the operations further comprise:
when it is predicted that the data input is not a sample from the distribution, screening the data input from input to the machine-learned classifier model.

5. The computing system of any preceding claim, wherein one or both of the machine-learned generative semantic model and the machine-learned generative background model comprise a recurrent neural network or a convolutional neural network.

6. The computing system of any preceding claim, wherein:

each of the plurality of in-distribution training examples comprises portions relevant for classifying the corresponding example into one of a plurality of in-distribution classes associated with the set of in-distribution training data; and
the perturbation of the one or more in-distribution training examples to generate the one or more background training examples corrupts said portions included in the one or more in-distribution training examples.

7. The computing system of any preceding claim, wherein the machine-learned generative semantic model has been trained using a first loss function and the machine-learned generative background model has been trained using a second loss function that comprises the first loss function with an additional L2 regularization term added; and/or wherein the machine-learned generative semantic model has been trained for a first number of iterations and the machine-learned generative background model has been trained using a second number of iterations that is less than the first number of iterations.

8. A computer-implemented method to perform genomic sequence classification for bacterial identification or medical diagnosis, the method comprising:

obtaining (302), by one or more computing devices, a set of in-distribution training data comprising a plurality of in-distribution training examples;
training (304), by the one or more computing devices, a machine-learned generative semantic model (202) using the set of in-distribution training data;
perturbing (306), by the one or more computing devices, one or more in-distribution training examples of the plurality of in-distribution training examples to generate one or more background training examples;
training (308), by the one or more computing devices, a machine-learned generative background model (203) using a set of background training data that comprises the one or more background training examples;
inputting, by the one or more computing devices, a data input into the machine-learned generative semantic model that has been trained on the set of in-distribution training data;
receiving, by the one or more computing devices, a first likelihood value (206) for the data input as an output of the machine-learned generative semantic model;
inputting, by the one or more computing devices, the data input into the machine-learned generative background model that has been trained on the set of background training data;
receiving, by the one or more computing devices, a second likelihood value (208) for the data input as an output of the machine-learned generative background model;
determining, by the one or more computing devices, a likelihood ratio value (210) for the data input based at least in part on the first likelihood value generated by the machine-learned generative semantic model and the second likelihood value generated by the machine-learned generative background model;
predicting whether the data input is not a sample from the distribution (212) based at least in part on the likelihood ratio value; and
when it is predicted that the data input is a sample from the distribution, providing the data input to a machine-learned classifier model for classification relative to a plurality of in-distribution classes;

wherein the data input is a genomic sequence, and
each of the one or more in-distribution training examples comprises a respective genomic sequence of DNA characters and perturbing, by the one or more computing devices, the one or more in-distribution training examples of the plurality of in-distribution training examples to generate the one or more background training examples comprises randomly mutating, by the computing device for each of the one or more in-distribution training examples, one or more characters of the respective genomic sequence of DNA characters to alternative DNA characters.

9. A computer-implemented method to perform image classification, the method comprising:

obtaining (302), by one or more computing devices, a set of in-distribution training data comprising a plurality of in-distribution training examples;
training (304), by the one or more computing devices, a machine-learned generative semantic model (202) using the set of in-distribution training data;
perturbing (306), by the one or more computing devices, one or more in-distribution training examples of the plurality of in-distribution training examples to generate one or more background training examples;
training (308), by the one or more computing devices, a machine-learned generative background model (203) using a set of background training data that comprises the one or more background training examples;
inputting, by the one or more computing devices, a data input into the machine-learned generative semantic model that has been trained on the set of in-distribution training data;
receiving, by the one or more computing devices, a first likelihood value (206) for the data input as an output of the machine-learned generative semantic model;
inputting, by the one or more computing devices, the data input into the machine-learned generative background model that has been trained on the set of background training data;
receiving, by the one or more computing devices, a second likelihood value (208) for the data input as an output of the machine-learned generative background model;
determining, by the one or more computing devices, a likelihood ratio value (210) for the data input based at least in part on the first likelihood value generated by the machine-learned generative semantic model and the second likelihood value generated by the machine-learned generative background model;
predicting whether the data input is not a sample from the distribution (212) based at least in part on the likelihood ratio value; and
when it is predicted that the data input is a sample from the distribution, providing the data input to a machine-learned classifier model for classification relative to a plurality of in-distribution classes;
wherein the data input is an image, and
each of the one or more in-distribution training examples comprises a respective image that comprises a plurality of pixels and perturbing, by the one or more computing devices, the one or more in-distribution training examples of the plurality of in-distribution training examples to generate the one or more background training examples comprises randomly changing, by the computing device for each of the one or more in-distribution training examples, one or more pixel values of the respective image to an alternative values.

10. The computer-implemented method of claim 8 or 9, wherein determining, by the one or more computing devices, the likelihood ratio (210) for the data input comprises determining, by the one or more computing devices, a logarithm of the first likelihood value (206) divided by the second likelihood value (208); and/or

wherein predicting, by the one or more computing devices, whether the data input is not a sample from the distribution based at least in part on the likelihood ratio value (210) comprises:
comparing, by the one or more computing devices, the likelihood ratio value to a threshold value; and
predicting, by the one or more computing devices, that the data input is not a sample from the distribution when the likelihood ratio value is less than the threshold value.

11. The computer-implemented method of any of claims 8-10, further comprising:
when it is predicted that the data input is a sample from the distribution, screening, by the one or more computing devices, the data input from input to the machine-learned classifier model.

12. The computer-implemented method of any of claims 8-11, wherein one or both of the machine-learned generative semantic model and the machine-learned generative background model comprise either a recurrent neural network, a convolutional neural network or an auto-regressive model.

**13.** The computer-implemented method of any of claims 8-12, wherein:

each of the plurality of in-distribution training examples comprises portions relevant for classifying the corresponding example into one of a plurality of in-distribution classes associated with the set of in-distribution training data; and

perturbing, by the one or more computing devices, the one or more in-distribution training examples of the plurality of in-distribution training examples to generate the one or more background training examples comprises corrupting, by the one or more computing devices, said portions included in each of the one or more in-distribution training examples.

**14.** The computer-implemented method of any of claims 8-13, wherein:

training, by the one or more computing devices, the machine-learned generative semantic model comprises training, by the one or more computing devices, the machine-learned generative semantic model using a first loss function; and

training, by the one or more computing devices, the machine-learned generative background model comprises training, by the one or more computing devices, the machine-learned generative background model using a second loss function that comprises the first loss function with an additional L2 regularization term added; and/or wherein:

training, by the one or more computing devices, the machine-learned generative semantic model comprises training, by the one or more computing devices, the machine-learned generative semantic model for a first number of training iterations; and

training, by the one or more computing devices, the machine-learned generative background model comprises training, by the one or more computing devices, the machine-learned generative background model for a second number of training iterations that is less than the first number of training iterations.

**15.** One or more non-transitory computer-readable media that collectively store instructions that, when executed by one or more computing devices, cause the one or more computing devices to perform the methods described in any of claims 8-14.


**Patentansprüche**

**1.** Computersystem, das Genomsequenzklassifizierung zur Bakterienidentifizierung oder medizinischen Diagnose durchführt, wobei Computersystem umfasst:

einen oder mehrere Prozessoren; und
ein oder mehrere nicht-transitorische computerlesbare Medien, die zusammen speichern:

ein maschinengelerntes generatives semantisches Modell (202), trainiert an einem Satz von In-Distribution-Trainingsdaten, die eine Vielzahl von In-Distribution-Trainingsbeispielen umfassen, welche Stichproben einer Verteilung sind, wobei das maschinengelernte generative semantische Modell konfiguriert ist, eine Dateneingabe zu empfangen und zu verarbeiten, um einen ersten Wahrscheinlichkeitswert (206) für die Dateneingabe zu erzeugen, wobei der erste Wahrscheinlichkeitswert eine erste Angabe der Wahrscheinlichkeit ist, dass die Dateneingabe eine Stichprobe aus der Verteilung ist;
ein maschinengelerntes generatives Hintergrundmodell (203), trainiert an einem Satz von Hintergrund-Trainingsdaten, die eine Vielzahl von Hintergrund-Trainingsbeispielen umfassen, wobei ein oder mehrere Hintergrund-Trainingsbeispiele der Vielzahl von Hintergrund-Trainingsbeispielen durch Störung eines oder mehrerer In-Distribution-Trainingsbeispiele der Vielzahl von In-Distribution-Trainingsbeispielen (306) erzeugt werden, wobei das maschinengelernte generative Hintergrundmodell konfiguriert ist, die Dateneingabe zu empfangen und zu verarbeiten, um einen zweiten Wahrscheinlichkeitswert (208) für die Dateneingabe zu erzeugen, wobei der zweite Wahrscheinlichkeitswert eine zweite Angabe der Wahrscheinlichkeit ist, dass die Dateneingabe eine Stichprobe aus einer Hintergrundverteilung ist; und
Anweisungen, die, wenn sie von dem einen oder den mehreren Prozessoren ausgeführt werden, Computersystem veranlassen, Operationen durchzuführen, umfassend:

Bestimmen eines Wahrscheinlichkeitsverhältniswertes (210) für die Dateneingabe basierend wenigstens teilweise auf dem erstem Wahrscheinlichkeitswert, der von dem maschinengelernte generativen semantischen Modell erzeugt wird, und dem zweitem Wahrscheinlichkeitswert, der von dem maschinengelernten generativen Hintergrundmodell erzeugt wird;

Vorhersagen, ob die Dateneingabe keine Stichprobe aus der Verteilung ist (212), basierend wenigstens teilweise auf dem Wahrscheinlichkeitsverhältniswert; und

wenn vorhergesagt wird, dass die Dateneingabe eine Stichprobe aus der Verteilung ist, Bereitstellen der Dateneingabe an ein maschinengelerntes Klassifikatormodell zur Klassifizierung relativ zu einer Vielzahl von In-Distribution-Klassen;

wobei die Dateneingabe eine Genomsequenz ist, und

jedes des einen oder der mehreren In-Distribution-Trainingsbeispiele eine jeweilige Genomsequenz von DNA-Zeichen umfasst und Störung des einen oder der mehreren In-Distribution-Trainingsbeispiele zur Erzeugung des einen oder der mehreren Hintergrund-Trainingsbeispiele das zufällige Mutieren eines oder mehrerer Zeichen jeder jeweiligen Genomsequenz von DNA-Zeichen zu einem oder mehreren alternativen DNA-Zeichen umfasst.

2. Computersystem, das Bildklassifizierung durchführt, wobei Computersystem umfasst:

einen oder mehrere Prozessoren; und
ein oder mehrere nicht-transitorische computerlesbare Medien, die zusammen speichern:

maschinengelerntes generatives semantisches Modell (202), trainiert an einem Satz von In-Distribution-Trainingsdaten, die eine Vielzahl von In-Distribution-Trainingsbeispielen umfassen, welche Stichproben einer Verteilung sind, wobei maschinengelerntes generatives semantisches Modell konfiguriert ist, Dateneingabe zu empfangen und zu verarbeiten, um ersten Wahrscheinlichkeitswert (206) für Dateneingabe zu erzeugen, wobei erster Wahrscheinlichkeitswert erste Angabe der Wahrscheinlichkeit ist, dass Dateneingabe eine Stichprobe aus Verteilung ist;

maschinengelerntes generatives Hintergrundmodell (203), trainiert an einem Satz von Hintergrund-Trainingsdaten, die eine Vielzahl von Hintergrund-Trainingsbeispielen umfassen, wobei ein oder mehrere Hintergrund-Trainingsbeispiele der Vielzahl von Hintergrund-Trainingsbeispielen durch Störung eines oder mehrerer In-Distribution-Trainingsbeispiele der Vielzahl von In-Distribution-Trainingsbeispielen (306) erzeugt werden, wobei maschinengelerntes generatives Hintergrundmodell konfiguriert ist, Dateneingabe zu empfangen und zu verarbeiten, um zweiten Wahrscheinlichkeitswert (208) für Dateneingabe zu erzeugen, wobei zweiter Wahrscheinlichkeitswert zweite Angabe der Wahrscheinlichkeit ist, dass Dateneingabe eine Stichprobe aus einer Hintergrundverteilung ist; und

Anweisungen, die, wenn sie von dem einen oder den mehreren Prozessoren ausgeführt werden, Computersystem veranlassen, Operationen durchzuführen, umfassend:

Bestimmen eines Wahrscheinlichkeitsverhältniswertes (210) für Dateneingabe basierend wenigstens teilweise auf erstem Wahrscheinlichkeitswert, der von maschinengelerntem generativen semantischen Modell erzeugt wird, und zweitem Wahrscheinlichkeitswert, der von maschinengelerntem generativen Hintergrundmodell erzeugt wird;

Vorhersagen, ob Dateneingabe keine Stichprobe aus Verteilung ist (212), basierend wenigstens teilweise auf Wahrscheinlichkeitsverhältniswert; und

wenn vorhergesagt wird, dass Dateneingabe eine Stichprobe aus Verteilung ist, Bereitstellen der Dateneingabe an maschinengelerntes Klassifikatormodell zur Klassifizierung relativ zu einer Vielzahl von In-Distribution-Klassen;

wobei Dateneingabe ein Bild ist, und

jedes des einen oder der mehreren In-Distribution-Trainingsbeispiele ein jeweiliges Bild umfasst, das eine Vielzahl von Pixeln umfasst, und Störung des einen oder der mehreren In-Distribution-Trainingsbeispiele zur Erzeugung des einen oder der mehreren Hintergrund-Trainingsbeispiele für jedes jeweilige Bild das zufällige Ändern jeweiliger Pixelwerte für eines oder mehrere der Vielzahl von Pixeln umfasst.

3. Computersystem nach Anspruch 1 oder 2, wobei Bestimmen des Wahrscheinlichkeitsverhältniswertes (210) für Dateneingabe Bestimmen eines Logarithmus des ersten Wahrscheinlichkeitswertes (206) geteilt durch zweiten Wahrscheinlichkeitswert (208) umfasst; und/oder

wobei Vorhersagen, ob Dateneingabe keine Stichprobe aus Verteilung ist, basierend wenigstens teilweise auf Wahrscheinlichkeitsverhältniswert (210), umfasst:

Vergleichen des Wahrscheinlichkeitsverhältniswertes mit einem Schwellenwert; und

Vorhersagen, dass Dateneingabe keine Stichprobe aus Verteilung ist, wenn Wahrscheinlichkeitsverhältniswert kleiner als Schwellenwert ist.

4. Computersystem nach einem der vorhergehenden Ansprüche, wobei Operationen ferner umfassen:

wenn vorhergesagt wird, dass Dateneingabe keine Stichprobe aus Verteilung ist, Ausfiltern der Dateneingabe vom Input für maschinengelerntes Klassifikatormodell.

5. Computersystem nach einem der vorhergehenden Ansprüche, wobei eines oder beide des maschinengelernten generativen semantischen Modells und des maschinengelernten generativen Hintergrundmodells ein rekurrentes neuronales Netzwerk oder ein konvolutionelles neuronales Netzwerk umfassen.

6. Computersystem nach einem der vorhergehenden Ansprüche, wobei:

jedes der Vielzahl von In-Distribution-Trainingsbeispielen Abschnitte umfasst, die für Klassifizierung des entsprechenden Beispiels in eine von einer Vielzahl von In-Distribution-Klassen, die dem Satz von In-Distribution-Trainingsdaten zugeordnet sind, relevant sind; und

Störung des einen oder der mehreren In-Distribution-Trainingsbeispiele zur Erzeugung des einen oder der mehreren Hintergrund-Trainingsbeispiele die Abschnitte, die in dem einen oder den mehreren In-Distribution-Trainingsbeispielen enthalten sind, beschädigt.

7. Computersystem nach einem der vorhergehenden Ansprüche, wobei maschinengelerntes generatives semantisches Modell unter Verwendung einer ersten Verlustfunktion trainiert wurde und maschinengelerntes generatives Hintergrundmodell unter Verwendung einer zweiten Verlustfunktion trainiert wurde, die die erste Verlustfunktion mit einem zusätzlichen L2-Regularisierungsterm umfasst; und/oder

wobei maschinengelerntes generatives semantisches Modell für eine erste Anzahl von Iterationen trainiert wurde und maschinengelerntes generatives Hintergrundmodell unter Verwendung einer zweiten Anzahl von Iterationen trainiert wurde, die kleiner als erste Anzahl von Iterationen ist.

8. Computerimplementiertes Verfahren zur Genomsequenzklassifizierung für Bakterienidentifizierung oder medizinische Diagnose, wobei Verfahren umfasst:

Erlangen (302), durch eine oder mehrere Computervorrichtungen, eines Satzes von In-Distribution-Trainingsdaten, die eine Vielzahl von In-Distribution-Trainingsbeispielen umfassen;

Trainieren (304), durch die eine oder die mehreren Computervorrichtungen, eines maschinengelernten generativen semantischen Modells (202) unter Verwendung des Satzes von In-Distribution-Trainingsdaten;

Stören (306), durch die eine oder die mehreren Computervorrichtungen, eines oder mehrerer In-Distribution-Trainingsbeispiele der Vielzahl von In-Distribution-Trainingsbeispielen, um ein oder mehrere Hintergrund-Trainingsbeispiele zu erzeugen;

Trainieren (308), durch die eine oder die mehreren Computervorrichtungen, eines maschinengelernten generativen Hintergrundmodells (203) unter Verwendung eines Satzes von Hintergrund-Trainingsdaten, der das eine oder die mehreren Hintergrund-Trainingsbeispiele umfasst;

Eingeben, durch die eine oder die mehreren Computervorrichtungen, einer Dateneingabe in maschinengelerntes generatives semantisches Modell, das an dem Satz von In-Distribution-Trainingsdaten trainiert wurde;

Empfangen, durch die eine oder die mehreren Computervorrichtungen, eines ersten Wahrscheinlichkeitswertes (206) für Dateneingabe als eine Ausgabe des maschinengelernten generativen semantischen Modells;

Eingeben, durch die eine oder die mehreren Computervorrichtungen, der Dateneingabe in maschinengelerntes generatives Hintergrundmodell, das an dem Satz von Hintergrund-Trainingsdaten trainiert wurde;

Empfangen, durch die eine oder die mehreren Computervorrichtungen, eines zweiten Wahrscheinlichkeitswertes (208) für Dateneingabe als eine Ausgabe des maschinengelernten generativen Hintergrundmodells;

Bestimmen, durch die eine oder die mehreren Computervorrichtungen, eines Wahrscheinlichkeitsverhältniswertes (210) für Dateneingabe basierend wenigstens teilweise auf erstem Wahrscheinlichkeitswert, der von maschinengelerntem generativen semantischen Modell erzeugt wird, und zweitem Wahrscheinlichkeitswert, der von maschinengelerntem generativen Hintergrundmodell erzeugt wird;

Vorhersagen, ob Dateneingabe keine Stichprobe aus Verteilung ist (212), basierend wenigstens teilweise auf Wahrscheinlichkeitsverhältniswert; und

wenn vorhergesagt wird, dass Dateneingabe eine Stichprobe aus Verteilung ist, Bereitstellen der Dateneingabe an maschinengelerntes Klassifikatormodell zur Klassifizierung relativ zu einer Vielzahl von In-Distribution-Klassen;

wobei Dateneingabe eine Genomsequenz ist, und

jedes des einen oder der mehreren In-Distribution-Trainingsbeispiele eine jeweilige Genomsequenz von DNA-Zeichen umfasst und Stören, durch die eine oder die mehreren Computervorrichtungen, des einen oder der mehreren In-Distribution-Trainingsbeispiele der Vielzahl von In-Distribution-Trainingsbeispielen zur Erzeugung des einen oder der mehreren Hintergrund-Trainingsbeispiele das zufällige Mutieren, durch Computervorrichtung für jedes des einen oder der mehreren In-Distribution-Trainingsbeispiele, eines oder mehrerer Zeichen der jeweiligen Genomsequenz von DNA-Zeichen zu alternativen DNA-Zeichen umfasst.

9. Computerimplementiertes Verfahren zum Durchführen von Bildklassifizierung, wobei Verfahren umfasst:

Erlangen (302), durch eine oder mehrere Computervorrichtungen, eines Satzes von In-Distribution-Trainingsdaten, die eine Vielzahl von In-Distribution-Trainingsbeispielen umfassen;

Trainieren (304), durch die eine oder die mehreren Computervorrichtungen, eines maschinengelernten generativen semantischen Modells (202) unter Verwendung des Satzes von In-Distribution-Trainingsdaten;

Stören (306), durch die eine oder die mehreren Computervorrichtungen, eines oder mehrerer In-Distribution-Trainingsbeispiele der Vielzahl von In-Distribution-Trainingsbeispielen, um ein oder mehrere Hintergrund-Trainingsbeispiele zu erzeugen;

Trainieren (308), durch die eine oder die mehreren Computervorrichtungen, eines maschinengelernten generativen Hintergrundmodells (203) unter Verwendung eines Satzes von Hintergrund-Trainingsdaten, der das eine oder die mehreren Hintergrund-Trainingsbeispiele umfasst;

Eingeben, durch die eine oder die mehreren Computervorrichtungen, einer Dateneingabe in maschinengelerntes generatives semantisches Modell, das an dem Satz von In-Distribution-Trainingsdaten trainiert wurde;

Empfangen, durch die eine oder die mehreren Computervorrichtungen, eines ersten Wahrscheinlichkeitswertes (206) für Dateneingabe als eine Ausgabe des maschinengelernten generativen semantischen Modells;

Eingeben, durch die eine oder die mehreren Computervorrichtungen, der Dateneingabe in maschinengelerntes generatives Hintergrundmodell, das an dem Satz von Hintergrund-Trainingsdaten trainiert wurde;

Empfangen, durch die eine oder die mehreren Computervorrichtungen, eines zweiten Wahrscheinlichkeitswertes (208) für Dateneingabe als eine Ausgabe des maschinengelernten generativen Hintergrundmodells;

Bestimmen, durch die eine oder die mehreren Computervorrichtungen, eines Wahrscheinlichkeitsverhältniswertes (210) für Dateneingabe basierend wenigstens teilweise auf erstem Wahrscheinlichkeitswert, der von maschinengelerntem generativen semantischen Modell erzeugt wird, und zweitem Wahrscheinlichkeitswert, der von maschinengelerntem generativen Hintergrundmodell erzeugt wird;

Vorhersagen, ob Dateneingabe keine Stichprobe aus Verteilung ist (212), basierend wenigstens teilweise auf Wahrscheinlichkeitsverhältniswert; und

wenn vorhergesagt wird, dass Dateneingabe eine Stichprobe aus Verteilung ist, Bereitstellen der Dateneingabe an maschinengelerntes Klassifikatormodell zur Klassifizierung relativ zu einer Vielzahl von In-Distribution-Klassen;

wobei Dateneingabe ein Bild ist, und

jedes des einen oder der mehreren In-Distribution-Trainingsbeispiele ein jeweiliges Bild umfasst, das eine Vielzahl von Pixeln umfasst, und Stören, durch die eine oder die mehreren Computervorrichtungen, des einen oder der mehreren In-Distribution-Trainingsbeispiele der Vielzahl von In-Distribution-Trainingsbeispielen zur Erzeugung des einen oder der mehreren Hintergrund-Trainingsbeispiele das zufällige Ändern, durch Computervorrichtung für jedes des einen oder der mehreren In-Distribution-Trainingsbeispiele, eines oder mehrerer Pixelwerte des jeweiligen Bildes zu alternativen Werten umfasst.

10. Computerimplementiertes Verfahren nach Anspruch 8 oder 9, wobei Bestimmen, durch die eine oder die mehreren Computervorrichtungen, des Wahrscheinlichkeitsverhältnisses (210) für Dateneingabe Bestimmen, durch die eine oder die mehreren Computervorrichtungen, eines Logarithmus des ersten Wahrscheinlichkeitswertes (206) geteilt durch zweiten Wahrscheinlichkeitswert (208) umfasst; und/oder

wobei Vorhersagen, durch die eine oder die mehreren Computervorrichtungen, ob Dateneingabe keine Stichprobe aus Verteilung ist, basierend wenigstens teilweise auf Wahrscheinlichkeitsverhältniswert (210), umfasst:

Vergleichen, durch die eine oder die mehreren Computervorrichtungen, des Wahrscheinlichkeitsverhältniswertes mit einem Schwellenwert; und

Vorhersagen, durch die eine oder die mehreren Computervorrichtungen, dass Dateneingabe keine Stichprobe aus Verteilung ist, wenn Wahrscheinlichkeitsverhältniswert kleiner als Schwellenwert ist.

**11.** Computerimplementiertes Verfahren nach einem der Ansprüche 8-10, ferner umfassend:
wenn vorhergesagt wird, dass Dateneingabe eine Stichprobe aus Verteilung ist, Filtern, durch die eine oder die mehreren Computervorrichtungen, der Dateneingabe vom Input für maschinengelerntes Klassifikatormodell.

**12.** Computerimplementiertes Verfahren nach einem der Ansprüche 8-11, wobei eines oder beide des maschinengelernten generativen semantischen Modells und des maschinengelernten generativen Hintergrundmodells entweder ein rekurrentes neuronales Netzwerk, ein konvolutionelles neuronales Netzwerk oder ein autoregressives Modell umfassen.

**13.** Computerimplementiertes Verfahren nach einem der Ansprüche 8-12, wobei:

jedes der Vielzahl von In-Distribution-Trainingsbeispielen Abschnitte umfasst, die für Klassifizierung des entsprechenden Beispiels in eine von einer Vielzahl von In-Distribution-Klassen, die dem Satz von In-Distribution-Trainingsdaten zugeordnet sind, relevant sind; und
Stören, durch die eine oder die mehreren Computervorrichtungen, des einen oder der mehreren In-Distribution-Trainingsbeispiele der Vielzahl von In-Distribution-Trainingsbeispielen zur Erzeugung des einen oder der mehreren Hintergrund-Trainingsbeispiele das Korrumpieren, durch die eine oder die mehreren Computervorrichtungen, der in jedem des einen oder der mehreren In-Distribution-Trainingsbeispiele enthaltenen Abschnitte umfasst.

**14.** Computerimplementiertes Verfahren nach einem der Ansprüche 8-13, wobei:

Trainieren, durch die eine oder die mehreren Computervorrichtungen, des maschinengelernten generativen semantischen Modells Trainieren, durch die eine oder die mehreren Computervorrichtungen, des maschinengelernten generativen semantischen Modells unter Verwendung einer ersten Verlustfunktion umfasst; und
Trainieren, durch die eine oder die mehreren Computervorrichtungen, des maschinengelernten generativen Hintergrundmodells Trainieren, durch die eine oder die mehreren Computervorrichtungen, des maschinengelernten generativen Hintergrundmodells unter Verwendung einer zweiten Verlustfunktion umfasst, die erste Verlustfunktion mit einem zusätzlichen L2-Regularisierungsterm umfasst; und/oder wobei:

Trainieren, durch die eine oder die mehreren Computervorrichtungen, des maschinengelernten generativen semantischen Modells Trainieren, durch die eine oder die mehreren Computervorrichtungen, des maschinengelernten generativen semantischen Modells für eine erste Anzahl von Trainingsiterationen umfasst; und
Trainieren, durch die eine oder die mehreren Computervorrichtungen, des maschinengelernten generativen Hintergrundmodells Trainieren, durch die eine oder die mehreren Computervorrichtungen, des maschinengelernten generativen Hintergrundmodells für eine zweite Anzahl von Trainingsiterationen umfasst, die kleiner als erste Anzahl von Trainingsiterationen ist.

**15.** Ein oder mehrere nicht-transitorische computerlesbare Medien, die zusammen Anweisungen speichern, die, wenn sie von einer oder mehreren Computervorrichtungen ausgeführt werden, eine oder mehrere Computervorrichtungen veranlassen, die in einem der Ansprüche 8-14 beschriebenen Verfahren durchzuführen.

**Revendications**

**1.** Système informatique qui effectue une classification de séquence génomique pour l'identification bactérienne ou le diagnostic médical, le système informatique comprenant :

un ou plusieurs processeurs ; et
un ou plusieurs supports non transitoires lisibles par ordinateur qui stockent collectivement :

un modèle sémantique génératif appris par machine (202) formé sur un ensemble de données de formation en distribution comprenant une pluralité d'exemples de formation en distribution qui sont des échantillons d'une distribution, le modèle sémantique génératif appris par machine étant configuré pour recevoir et traiter une entrée de données pour générer une première valeur de vraisemblance (206) pour l'entrée de données, la première valeur de vraisemblance étant une première indication de la vraisemblance que l'entrée de données soit un échantillon de la distribution ;

un modèle d'arrière-plan génératif appris par machine (203) formé sur un ensemble de données de formation en arrière-plan comprenant une pluralité d'exemples de formation en arrière-plan, un ou plusieurs exemples de formation en arrière-plan de la pluralité d'exemples de formation en arrière-plan générés à travers la perturbation d'un ou de plusieurs exemples de formation en distribution de la pluralité d'exemples de formation en distribution (306), le modèle d'arrière-plan génératif appris par machine étant configuré pour recevoir et traiter l'entrée de données afin de générer une seconde valeur de vraisemblance (208) pour l'entrée de données, la seconde valeur de vraisemblance étant une seconde indication de la vraisemblance que l'entrée de données soit un échantillon d'une distribution d'arrière-plan ; et

des instructions qui, lorsqu'elles sont exécutées par l'un ou les plusieurs processeurs, amènent le système informatique à effectuer des opérations comprenant :

la détermination d'une valeur de rapport de vraisemblance (210) pour l'entrée de données sur la base au moins en partie de la première valeur de vraisemblance générée par le modèle sémantique génératif appris par machine et de la seconde valeur de vraisemblance générée par le modèle d'arrière-plan génératif appris par machine ;

la prédiction de savoir si l'entrée de données n'est pas un échantillon de la distribution (212) sur la base au moins en partie de la valeur du rapport de vraisemblance ; et

lorsqu'il est prédit que l'entrée de données est un échantillon de la distribution, la fourniture de l'entrée de données à un modèle de classificateur appris par machine pour la classification par rapport à une pluralité de classes en distribution ;

dans lequel l'entrée de données est une séquence génomique, et chacun de l'un ou des plusieurs exemples de formation en distribution comprend une séquence génomique respective de caractères d'ADN et la perturbation de l'un ou des plusieurs exemples de formation en distribution pour générer l'un ou les plusieurs exemples de formation en arrière-plan comprend la mutation aléatoire d'un ou de plusieurs caractères de chaque séquence génomique respective de caractères d'ADN en un ou plusieurs caractères d'ADN alternatifs.

2. Système informatique qui effectue la classification d'image, le système informatique comprenant :

un ou plusieurs processeurs ; et
un ou plusieurs supports non transitoires lisibles par ordinateur qui stockent collectivement :

un modèle sémantique génératif appris par machine (202) formé sur un ensemble de données de formation en distribution comprenant une pluralité d'exemples de formation en distribution qui sont des échantillons d'une distribution, le modèle sémantique génératif appris par machine étant configuré pour recevoir et traiter une entrée de données pour générer une première valeur de vraisemblance (206) pour l'entrée de données, la première valeur de vraisemblance étant une première indication de la vraisemblance que l'entrée de données soit un échantillon de la distribution ;

un modèle d'arrière-plan génératif appris par machine (203) formé sur un ensemble de données de formation en arrière-plan comprenant une pluralité d'exemples de formation en arrière-plan, un ou plusieurs exemples de formation en arrière-plan de la pluralité d'exemples de formation en arrière-plan générés à travers la perturbation d'un ou de plusieurs exemples de formation en distribution de la pluralité d'exemples de formation en distribution (306), le modèle d'arrière-plan génératif appris par machine étant configuré pour recevoir et traiter l'entrée de données afin de générer une seconde valeur de vraisemblance (208) pour l'entrée de données, la seconde valeur de vraisemblance étant une seconde indication de la vraisemblance que l'entrée de données soit un échantillon d'une distribution d'arrière-plan ; et

des instructions qui, lorsqu'elles sont exécutées par l'un ou les plusieurs processeurs, amènent le système informatique à effectuer des opérations comprenant :

la détermination d'une valeur de rapport de vraisemblance (210) pour l'entrée de données sur la base au moins en partie de la première valeur de vraisemblance générée par le modèle sémantique génératif appris par machine et de la seconde valeur de vraisemblance générée par le modèle d'arrière-plan génératif appris par machine ;

la prédiction de savoir si l'entrée de données n'est pas un échantillon de la distribution (212) sur la base au moins en partie de la valeur du rapport de vraisemblance ; et

lorsqu'il est prédit que l'entrée de données est un échantillon de la distribution, la fourniture de l'entrée de données à un modèle de classificateur appris par machine pour la classification par rapport à une pluralité de classes en distribution ;

dans lequel l'entrée de données est une image, et

chacun de l'un ou des plusieurs exemples de formation en distribution comprend une image respective comprenant une pluralité de pixels et la perturbation de l'un ou des plusieurs exemples de formation en distribution pour générer l'un ou les plusieurs exemples de formation en arrière-plan comprend, pour chaque image respective, la modification aléatoire des valeurs de pixels respectives pour un ou plusieurs de la pluralité de pixels.

3. Système informatique selon la revendication 1 ou 2, dans lequel la détermination de la valeur du rapport de vraisemblance (210) pour l'entrée de données comprend la détermination d'un logarithme de la première valeur de vraisemblance (206) divisé par la seconde valeur de vraisemblance (208) ; et/ou

dans lequel la prédiction de savoir si l'entrée de données n'est pas un échantillon de la distribution sur la base au moins en partie de la valeur du rapport de vraisemblance (210) comprend :

la comparaison de la valeur du rapport de vraisemblance à une valeur seuil ; et

la prédiction selon laquelle l'entrée de données n'est pas un échantillon de la distribution lorsque la valeur du rapport de vraisemblance est inférieure à la valeur seuil.

4. Système informatique selon une quelconque revendication précédente, dans lequel les opérations comprennent également :

lorsqu'il est prédit que l'entrée de données n'est pas un échantillon de la distribution, le filtrage de l'entrée de données dès l'entrée vers le modèle de classificateur appris par machine.

5. Système informatique selon une quelconque revendication précédente, dans lequel l'un ou les deux parmi le modèle sémantique génératif appris par machine et le modèle d'arrière-plan génératif appris par machine comprennent un réseau neuronal récurrent ou un réseau neuronal convolutif.

6. Système informatique selon une quelconque revendication précédente, dans lequel :

chacun de la pluralité d'exemples de formation en distribution comprend des parties pertinentes pour classer l'exemple correspondant dans l'une d'une pluralité de classes en distribution associées à l'ensemble de données de formation en distribution ; et

la perturbation de l'un ou de plusieurs exemples de formation en distribution pour générer l'un ou les plusieurs exemples de formation en arrière-plan corrompt lesdites parties incluses dans l'un ou les plusieurs exemples de formation en distribution.

7. Système informatique selon une quelconque revendication précédente, dans lequel le modèle sémantique génératif appris par machine a été formé à l'aide d'une première fonction de perte, et le modèle d'arrière-plan génératif appris par machine a été formé à l'aide d'une seconde fonction de perte comprenant la première fonction de perte avec un terme de régularisation L2 supplémentaire ajouté ; et/ou

dans lequel le modèle sémantique génératif appris par machine a été formé pour un premier nombre d'itérations et le modèle d'arrière-plan génératif appris par machine a été formé à l'aide d'un second nombre d'itérations qui est inférieur au premier nombre d'itérations.

8. Procédé mis en œuvre par ordinateur pour effectuer une classification de séquence génomique pour l'identification bactérienne ou le diagnostic médical, le procédé comprenant :

l'obtention (302), par un ou plusieurs dispositifs informatiques, d'un ensemble de données de formation en distribution comprenant une pluralité d'exemples de formation en distribution ;

la formation (304), par l'un ou les plusieurs dispositifs informatiques, d'un modèle sémantique génératif appris par machine (202) à l'aide de l'ensemble de données de formation en distribution ;

la perturbation (306), par l'un ou les plusieurs dispositifs informatiques, d'un ou de plusieurs exemples de formation en distribution de la pluralité d'exemples de formation en distribution pour générer un ou plusieurs exemples de formation en arrière-plan ;

la formation (308), par l'un ou les plusieurs dispositifs informatiques, d'un modèle d'arrière-plan génératif appris par machine (203) à l'aide d'un ensemble de données de formation en arrière-plan qui comprend l'un ou les plusieurs exemples de formation en arrière-plan ;

l'entrée, par l'un ou les plusieurs dispositifs informatiques, d'une entrée de données dans le modèle sémantique génératif appris par machine qui a été formée sur l'ensemble de données de formation en distribution ;

la réception, par l'un ou les plusieurs dispositifs informatiques, d'une première valeur de vraisemblance (206) pour l'entrée de données en tant que sortie du modèle sémantique génératif appris par machine ;

l'entrée, par l'un ou les plusieurs dispositifs informatiques, de l'entrée de données dans le modèle d'arrière-plan génératif appris par machine qui a été formée sur l'ensemble de données de formation en arrière-plan ;

la réception, par l'un ou les plusieurs dispositifs informatiques, d'une seconde valeur de vraisemblance (208) pour l'entrée de données en tant que sortie du modèle d'arrière-plan génératif appris par machine ;

la détermination, par l'un ou les plusieurs dispositifs informatiques, d'une valeur de rapport de vraisemblance (210) pour l'entrée de données sur la base au moins en partie de la première valeur de vraisemblance générée par le modèle sémantique génératif appris par machine et de la seconde valeur de vraisemblance générée par le modèle d'arrière-plan génératif appris par machine ;

la prédiction de savoir si l'entrée de données n'est pas un échantillon de la distribution (212) sur la base au moins en partie de la valeur du rapport de vraisemblance ; et

lorsqu'il est prédit que l'entrée de données est un échantillon de la distribution, la fourniture de l'entrée de données à un modèle de classificateur appris par machine pour la classification par rapport à une pluralité de classes en distribution ;

dans lequel l'entrée de données est une séquence génomique, et

chacun de l'un ou des plusieurs exemples de formation en distribution comprend une séquence génomique respective de caractères d'ADN et la perturbation, par l'un ou les plusieurs dispositifs informatiques, de l'un ou des plusieurs exemples de formation en distribution de la pluralité d'exemples de formation en distribution pour générer l'un ou les plusieurs exemples de formation en arrière-plan comprend la mutation aléatoire, par le dispositif informatique pour chacun de l'un ou des plusieurs exemples de formation en distribution, d'un ou de plusieurs caractères de la séquence génomique respective de caractères d'ADN en caractères d'ADN alternatifs.

9. Procédé mis en œuvre par ordinateur pour effectuer une classification d'image, le procédé comprenant :

l'obtention (302), par un ou plusieurs dispositifs informatiques, d'un ensemble de données de formation en distribution comprenant une pluralité d'exemples de formation en distribution ;

la formation (304), par l'un ou les plusieurs dispositifs informatiques, d'un modèle sémantique génératif appris par machine (202) à l'aide de l'ensemble de données de formation en distribution ;

la perturbation (306), par l'un ou les plusieurs dispositifs informatiques, d'un ou de plusieurs exemples de formation en distribution de la pluralité d'exemples de formation en distribution pour générer un ou plusieurs exemples de formation en arrière-plan ;

la formation (308), par l'un ou les plusieurs dispositifs informatiques, d'un modèle d'arrière-plan génératif appris par machine (203) à l'aide d'un ensemble de données de formation en arrière-plan qui comprend l'un ou les plusieurs exemples de formation en arrière-plan ;

l'entrée, par l'un ou les plusieurs dispositifs informatiques, d'une entrée de données dans le modèle sémantique génératif appris par machine qui a été formée sur l'ensemble de données de formation en distribution ;

la réception, par l'un ou les plusieurs dispositifs informatiques, d'une première valeur de vraisemblance (206) pour l'entrée de données en tant que sortie du modèle sémantique génératif appris par machine ;

l'entrée, par l'un ou les plusieurs dispositifs informatiques, de l'entrée de données dans le modèle d'arrière-plan génératif appris par machine qui a été formée sur l'ensemble de données de formation en arrière-plan ;

la réception, par l'un ou les plusieurs dispositifs informatiques, d'une seconde valeur de vraisemblance (208) pour l'entrée de données en tant que sortie du modèle d'arrière-plan génératif appris par machine ;

la détermination, par l'un ou les plusieurs dispositifs informatiques, d'une valeur de rapport de vraisemblance (210) pour l'entrée de données sur la base au moins en partie de la première valeur de vraisemblance générée par le modèle sémantique génératif appris par machine et de la seconde valeur de vraisemblance générée par le modèle d'arrière-plan génératif appris par machine ;

la prédiction de savoir si l'entrée de données n'est pas un échantillon de la distribution (212) sur la base au moins en partie de la valeur du rapport de vraisemblance ; et

lorsqu'il est prédit que l'entrée de données est un échantillon de la distribution, la fourniture de l'entrée de données à un modèle de classificateur appris par machine pour la classification par rapport à une pluralité de classes en distribution ;

dans lequel l'entrée de données est une image, et

chacun de l'un ou des plusieurs exemples de formation en distribution comprend une image respective qui comprend une pluralité de pixels et la perturbation, par l'un ou les plusieurs dispositifs informatiques, de l'un ou des plusieurs exemples de formation en distribution de la pluralité d'exemples de formation en distribution pour générer l'un ou les plusieurs exemples de formation en arrière-plan comprend la modification aléatoire, par le dispositif informatique pour chacun de l'un ou des plusieurs exemples de formation en distribution, d'une ou de plusieurs valeurs de pixels de l'image respective en valeurs alternatives.

**10.** Procédé mis en œuvre par ordinateur selon la revendication 8 ou 9, dans lequel la détermination, par l'un ou les plusieurs dispositifs informatiques, du rapport de vraisemblance (210) pour l'entrée de données comprend la détermination, par l'un ou les plusieurs dispositifs informatiques, d'un logarithme de la première valeur de vraisemblance (206) divisé par la seconde valeur de vraisemblance (208) ; et/ou

dans lequel la prédiction, par l'un ou les plusieurs dispositifs informatiques, de savoir si l'entrée de données n'est pas un échantillon de la distribution sur la base au moins en partie de la valeur du rapport de vraisemblance (210) comprend :

la comparaison, par l'un ou les plusieurs dispositifs informatiques, de la valeur du rapport de vraisemblance à une valeur seuil ; et

la prédiction, par l'un ou les plusieurs dispositifs informatiques, selon laquelle l'entrée de données n'est pas un échantillon de la distribution lorsque la valeur du rapport de vraisemblance est inférieure à la valeur seuil.

**11.** Procédé mis en œuvre par ordinateur selon l'une quelconque des revendications 8 à 10, comprenant également :
lorsqu'il est prédit que l'entrée de données est un échantillon de la distribution, le filtrage, par l'un ou les plusieurs dispositifs informatique, de l'entrée de données dès l'entrée vers le modèle de classificateur appris par machine.

**12.** Procédé mis en œuvre par ordinateur selon l'une quelconque des revendications 8 à 11, dans lequel l'un ou les deux parmi le modèle sémantique génératif appris par machine et le modèle d'arrière-plan génératif appris par machine comprennent soit un réseau neuronal récurrent, un réseau neuronal convolutif ou un modèle auto-régressif.

**13.** Procédé mis en œuvre par ordinateur selon l'une quelconque des revendications 8 à 12, dans lequel :

chacun de la pluralité d'exemples de formation en distribution comprend des parties pertinentes pour classer l'exemple correspondant dans l'une d'une pluralité de classes en distribution associées à l'ensemble de données de formation en distribution ; et

la perturbation, par l'un ou les plusieurs dispositifs informatiques, de l'un ou des plusieurs exemples de formation en distribution de la pluralité d'exemples de formation en distribution pour générer l'un ou les plusieurs exemples de formation en arrière-plan comprend la corruption, par l'un ou les plusieurs dispositifs informatiques, desdites parties incluses dans chacun de l'un ou des plusieurs exemples de formation en distribution.

**14.** Procédé mis en œuvre par ordinateur selon l'une quelconque des revendications 8 à 13, dans lequel :

la formation, par l'un ou les plusieurs dispositifs informatiques, du modèle sémantique génératif appris par machine comprend la formation, par l'un ou les plusieurs dispositifs informatiques, du modèle sémantique génératif appris par machine à l'aide d'une première fonction de perte ; et

la formation, par l'un ou les plusieurs dispositifs informatiques, du modèle d'arrière-plan génératif appris par machine comprend la formation, par l'un ou les plusieurs dispositifs informatiques, du modèle d'arrière-plan génératif appris par machine à l'aide d'une seconde fonction de perte qui comprend la première fonction de perte avec un terme de régularisation L2 supplémentaire ajouté ; et/ou dans lequel :

la formation, par l'un ou les plusieurs dispositifs informatiques, du modèle sémantique génératif appris par machine comprend la formation, par l'un ou les plusieurs dispositifs informatiques, du modèle sémantique génératif appris par machine pour un premier nombre d'itérations de formation ; et

la formation, par l'un ou les plusieurs dispositifs informatiques, du modèle d'arrière-plan génératif appris par machine comprend la formation, par l'un ou les plusieurs dispositifs informatiques, du modèle d'arrière-plan génératif appris par machine pour un second nombre d'itérations de formation qui est inférieur au premier nombre d'itérations de formation.

**15.** Un ou plusieurs supports lisibles par ordinateur non transitoires qui stockent collectivement des instructions qui, lorsqu'elles sont exécutées par un ou plusieurs dispositifs informatiques, amènent l'un ou les plusieurs dispositifs informatiques à effectuer les procédés selon l'une quelconque des revendications 8 à 14.

Figure 1A

Figure 1B

**Computing Device** — 50

| Application 1 | Application 2 | ••• | Application N |

**Central Intelligence Layer**

| Model 1 | Model 2 | ••• | Model N |

**Central Device Data Layer**

| Sensor(s) | Context Manager | Device State | Additional Component(s) |

# Figure 1C

Figure 2

<u>300</u>

302 — Obtain a set of in-distribution training data that includes a plurality of in-distribution training examples

304 — Train a machine-learned generative semantic model using the set of in-distribution training data

306 — Perturb one or more in-distribution training examples to generate one or more background training examples

308 — Train a machine-learned generative background model using a set of background training data that includes the one or more background training examples

# Figure 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62857774

**Non-patent literature cited in the description**

- **ARNAB PODDAR et al.** Quality Measures for Speaker Verification with Short Utterances. *Arxiv.org*, 2019
- **DAN HENDRYCKS et al.** Deep Anomaly Detection with Outlier Exposure. *Arxiv.org*, 2018
- **ERIC NALISNICK et al.** Hybrid Models with Deep and Invertible Features. *Arxiv.org*, 2019
- **AKOSUA BUSIA et al.** A deep learning approach to pattern recognition for short DNA sequences. *Biorxiv.org*, 2019
- **AARON VAN DEN OORD et al.** Pixel Recurrent Neural Networks. *Arxiv.org*, 2016
- **JIE REN et al.** Likelihood Ratios for Out-of-distribution Detection. *arXiv: 1906.02845*